# EUROPEAN PATENT APPLICATION

(11) **EP 1 582 517 A1**
(43) Date of publication of application: **05.10.2005**
(21) Application number: 04701099.6
(22) Date of filing: 09.01.2004
(51) Int. Cl.: C07D 231/26, A61K 31/4152, A61P 7/00, A61P 9/00, A61P 25/00, A61P 29/00, A61P 35/00, A61P 43/00

(54) **BLOOD-BRAIN BARRIER DISRUPTION INHIBITORS**

(30) Priority: 10.01.2003 JP 2003004813
(71) Applicant: Mitsubishi Pharma Corporation, Osaka 541-0046 (JP)
(72) Inventor: KAWAKAMI Junichi, Iyakudaishukusha 5-202, Imizu-gun, Toyama 939- 0364 (JP)
(74) Representative: Sternagel, Fleischer, Godemeyer & Partner
(86) International application number: PCT/JP2004/000105
(87) International publication number: WO 2004/063167

(57) **Abstract**

An object of the present invention is to provide a blood-brain barrier disruption inhibitor. The present invention provides a blood-brain barrier disruption inhibitor which comprises as an active ingredient a pyrazolone derivative represented by the following formula (I) or a physiologically acceptable salt thereof, or a hydrate thereof or a solvate thereof: 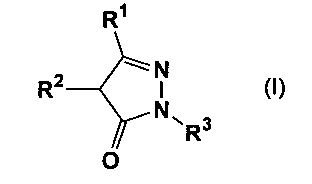 wherein R¹ represents a hydrogen atom, an aryl group, an alkyl group, or an alkoxycarbonylalkyl group; R² represents a hydrogen atom, an aryloxy group, an arylmercapto group, an alkyl group or a hydroxyalkyl group; or R¹ and R² are combined with each other to represent an alkylene group; and R³ represents a hydrogen atom, an alkyl group, a cycloalkyl group, a hydroxyalkyl group, a benzyl group, a naphthyl group, a phenyl group, or a phenyl group substituted with 1 to 3 substituents selected from the group consisting of an alkyl group, an alkoxy group, a hydroxyalkyl group, an alkoxycarbonyl group, an alkylmercapto group, an alkylamino group, a dialkylamino group, a halogen atom, a trifluoromethyl group, a carboxyl group, a cyano group, a hydroxyl group, a nitro group, an amino group and an acetamide group.

## Description

### TECHNICAL FIELD

The present invention relates to a blood-brain barrier disruption inhibitor which comprise, as an active ingredient, a pyrazolone derivative or a physiologically acceptable salt thereof, or a hydrate thereof or a solvate thereof.

### BACKGROUND ART

The blood-brain barrier is a barrier for restricting the transfer of substances from the blood to brain tissues. The brain is protected from toxic substances with such a blood-brain barrier. Lipid-soluble substances such as nicotine, caffeine or heroin can easily pass through the blood-brain barrier. However, in general, lipid-insoluble substances such as a polar substance or a strong electrolyte can hardly pass through the blood-brain barrier. It has been known that water-soluble substances such as D-glucose, which are necessary for brain metabolism, permeate the blood-brain barrier via carriers, and they are then transferred to brain tissues. In brain capillaries, endothelial cells, which are adjacent to each other, form a tight bond that is called a tight junction, so that they prevent substances from leaking out of spaces between cells. Thus, it is considered that substances moving in and out of the brain should pass through such brain capillary endothelial cells, in principle. As stated above, brain capillary endothelial cells transport not only nutritive substances but also medicaments into the brain, through various transport systems that have expressed in the cytoplasmic membrane.

In the case of inflammatory diseases of central nervous system such as multiple sclerosis, meningitis, cerebritis or brain abscess, it has been reported that the blood-brain barrier is disrupted, and that TNF-α and IL-1β which are inflammatory cytokines existing in spinal fluid exhibits high values (S. L. Hauser, et al., Cytokine accumulation in CSF of multiple sclerosis patients: frequent detection of interleukin-1 and tumor necrosis factor but not interleukin-6. Neurology, 40: 1735-1739 (1990)).

Regarding a pyrazolone derivative which is represented by the following formula (I): 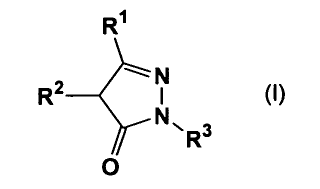 wherein R¹ represents a hydrogen atom, aryl, C₁₋₅ alkyl, or C₃₋₆ (total carbon number) alkoxycarbonylalkyl, R² represents a hydrogen atom, aryloxy, arylmercapto, C₁₋₅ alkyl or C₁₋₃ hydroxyalkyl, or R¹ and R² are combined with each other to represent C₃₋₅ alkylene group, and R³ represents a hydrogen atom, C₁₋₅ alkyl, C₅₋₇ cycloalkyl, C₁₋₃ hydroxyalkyl, benzyl, naphthyl or phenyl, or phenyl substituted with the same or different 1 to 3 substituents selected from the group consisting of C₁₋₅ alkoxy, C₁₋₃ hydroxyalkyl, C₂₋₅ (total carbon number) alkoxycarbonyl, C₁₋₃ alkylmercapto, C₁₋₄ alkylamino, C₂₋₈ (total carbon number) dialkylamino, halogen atom, trifluoromethyl, carboxyl, cyano, hydroxyl group, nitro, amino and acetamide), examples of the known medical applications include cerebral function-normalizing action (JP Patent Publication (Kokoku) No. 5-31523 B (1993)), lipid peroxide production-suppressing action (JP Patent Publication (Kokoku) No. 5-35128, B (1993), antiulcer action (JP Patent Publication (Kokai) No. 3-215425 (1991)) and anti-hyperglycemic action (JP Patent Publication (Kokai) No. 3-215426 (1991)).

Among the compounds represented by the above formula (I), a pharmaceutical preparation containing 3-methyl-1-phenyl-2-pyrazolin-5-one as an active ingredient has been commercially available as a protective agent for the brain (under the general name "edaravone" and the commercial name "Radicut": produced and marketed by Mitsubishi Pharma Corporation) since June 2001. This "edaravone" has been reported to have high reactivity to active oxygen (Kawai, H., et al., J. Phamacol. Exp. Ther., 281(2), 921, 1997; Wu, TW. et al., Life Sci, 67(19), 2387, 2000). As described above, edaravone is a free radical scavenger which prevents cell damage and the like by removing various free radicals including active oxygen. However, to date, there have been no reports regarding whether or not edaravone is able to inhibit disruption of the blood-brain barrier.

### DISCLOSURE OF THE INVENTION

It is an object of the present invention to provide a blood-brain barrier disruption inhibitor.

In order to achieve the aforementioned object, the present inventors have studied the effect of a pyrazolone derivative represented by formula (I) to inhibit blood-brain barrier disruption, using *in vitro* and *in vivo* systems. As a result, the inventors have found that blood-brain barrier disruption is inhibited by administration of the above-described pyrazolone derivative, so as to alleviate the neurological symptoms of patients suffering from inflammatory diseases of central nervous system, thereby completing the present invention.

According to the present invention, there is provided a blood-brain barrier disruption inhibitor which comprises as an active ingredient a pyrazolone derivative represented by the following formula (I) or a physiologically acceptable salt thereof, or a hydrate thereof or a solvate thereof: 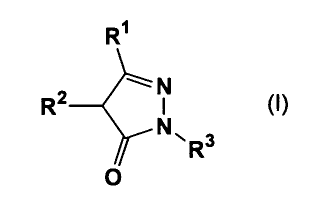 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

In a preferred aspect, the present invention provides a blood-brain barrier disruption inhibitor which has an action of inhibiting increases in permeability of the blood-brain barrier, and a blood-brain barrier disruption inhibitor which has an action of inhibiting increases in the amount of inflammatory cytokines in spinal fluid.

In a preferred aspect of the present invention, the pyrazolone derivative represented by the formula (I) is 3-methyl-1-phenyl-2-pyrazolin-5-one or a physiologically acceptable salt thereof, or a hydrate thereof or a solvate thereof.

In another aspect, the present invention provides a medicament for prevention and/or treatment of multiple sclerosis, meningitis, cerebritis or brain abscess, which comprises as an active ingredient a pyrazolone derivative represented by the above-described formula (I) or a physiologically acceptable salt thereof, or a hydrate thereof or a solvate thereof. According to a preferred aspect, the pyrazolone derivative represented by the formula (I) is 3-methyl-1-phenyl-2-pyrazolin-5-one.

In further another aspect, the present invention provides a method for inhibiting a blood-brain barrier disruption which comprises a step of administering to mammals such as a human, an effective amount of a pyrazolone derivative represented by the formula (I) or a physiologically acceptable salt thereof, or a hydrate thereof or a solvate thereof; and a method for preventing and/or treating multiple sclerosis, meningitis, cerebritis or brain abscess which comprises a step of administering to mammals such as a human, an effective amount of a pyrazolone derivative represented by the formula (I) or a physiologically acceptable salt thereof, or a hydrate thereof or a solvate thereof.

In another aspect, the present invention provides the use of a pyrazolone derivative represented by formula (I) or a physiologically acceptable salt thereof, or a hydrate thereof or a solvate thereof, for the production of a blood-brain barrier disruption inhibitor; and the use of a pyrazolone derivative represented by formula (I) or a physiologically acceptable salt thereof, or a hydrate thereof or a solvate thereof, for the production of a medicament for prevention and/or treatment of multiple sclerosis, meningitis, cerebritis or brain abscess.

### BRIEF DESCRIPTION OF THE DRAWINGS

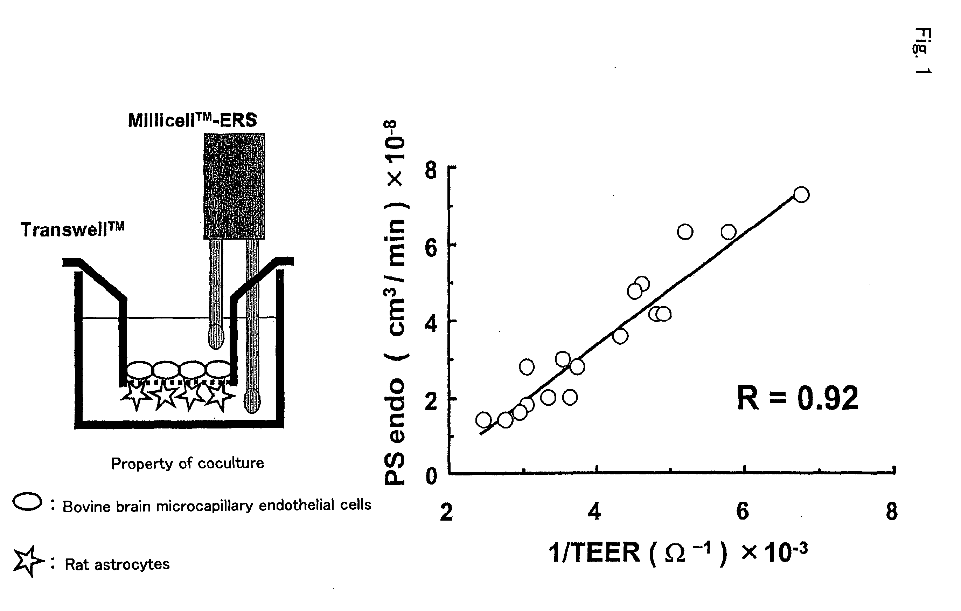

Figure 1 shows a summary of a blood-brain barrier coculture model (left figure), and the correlation between the diffusion speed of sodium fluorescein and TEER (transcellular endothelial electrical resistance) in the produced coculture model (right figure). In Figure 1, the term "PSendo" indicates a fluorescein permeation clearance in an endothelial cell layer. Fluorescein is added into a medium on the apical side (on the side of the endothelial cells) of a Transwell, and fluorescein permeation into the medium on the basolateral side (the side of astrocytes) is observed. The permeation clearance is obtained by dividing the amount of fluorescein that has permeated the basolateral side over a certain period of time by the concentration of fluorescein contained in the medium on the apical side. Since the permeation clearance is measured as the total permeation clearance (PStotal) consisting of the permeation clearance of an endothelial cell layer, that of a Transwell filter, and that of astrocytes in the experiment, the obtained value is corrected by using the permeation clearance obtained when only astrocytes are cultured in a Transwell filter (PSastro + filter). The correction formula is as follows: 1/PStotal = 1/PSendo + 1/PSastro + filter.

Figure 2 shows the results obtained by examining the effects of TNF-α (tumor necrosis factor alpha) and IL-1β (interleukin-1 beta) on TEER.

Figure 3 shows the effects of NF-κB (nuclear factor-κB) inhibitor and iNOS (inducible nitric oxide synthase) inhibitor on TEER. The term "α-MSH" indicates an alpha melanocyte-stimulating hormone.

Figure 4 shows the effects of NF-κB inhibitor and iNOS inhibitor on the amount of NO produced.

Figure 5 shows the effects of edaravone on TEER.

Figure 6 shows the results obtained by examining the amounts of TNF-α and IL-1β and the BBB permeability in EAE rats.

Figure 7 shows the results obtained by examining the effect of edaravone on the amounts of TNF-α and IL-1β and the BBB permeability in EAE rats. The term "CSF" indicates cerebral spinal fluid.

Figure 8 shows the results obtained by evaluating limb neuroparalysis scores in a group of EAE model rats to which no agents are administered (control), a group of EAE model rats to which dexamethasone is administered, and a group of EAE model rats to which edaravone is administered. The term "i.p." indicates intraperitoneal administration.

### BEST MODE FOR CARRYING OUT THE INVENTION

The blood-brain barrier disruption inhibitor and the medicament for prevention and/or treatment of multiple sclerosis, meningitis, cerebritis, or brain abscess according to the present invention (hereinafter referred to as "the agent of the present invention" at times) comprise a pyrazolone derivative represented by the formula (I) that is defined in the present specification or a physiologically acceptable salt thereof, or a hydrate thereof or a solvate thereof.

The compound represented by the formula (I) used in the present invention can have a structure represented by the following formula (I') or (I") due to tautomerism. One of the tautomers is shown in the formula (I) of this specification for convenience. The presence of the following tautomers is obvious to a person skilled in the art. As an active ingredient of the medicament of the present invention, the compound represented by the following formula (I') or (I") or a physiologically acceptable salt thereof, or a hydrate thereof or a solvate thereof may also be used.

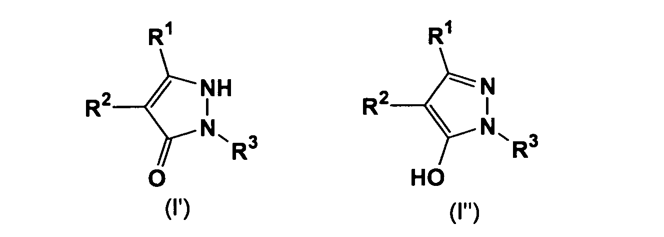

In the formula (I), the aryl group in the definition of R¹ may be either a monocyclic or polycyclic aryl group. Examples thereof include a phenyl group, a naphthyl group and the like, as well as a phenyl group substituted with a substituent such as an alkyl group (for example, a methyl group or a butyl group), an alkoxy group (for example, a methoxy group or a butoxy group), a halogen atom (for example, a chlorine atom) or a hydroxy group. The same applies for aryl portions in other substituents (e.g., an aryloxy group) having the aryl portions.

The C₁₋₅ alkyl group in the definition of R¹, R² and R³ may be either linear- or branched chain. Examples thereof include a methyl group, an ethyl group, a propyl group, an isopropyl group, a butyl group, an isobutyl group, a sec-butyl group, a tert-butyl group, and a pentyl group. The same applies for alkyl portions in other substituents (alkoxycarbonylalkyl group) having the alkyl portions.

Examples of the C₃₋₆ (total carbon number) alkoxycarbonylalkyl group in the definition of R¹ include a methoxycarbonylmethyl group, an ethoxycarbonylmethyl group, a propoxycarbonylmethyl group, a methoxycarbonylethyl group and a methoxycarbonylpropyl group.

Examples of the aryloxy group in the definition of R² include a p-methylphenoxy group, a p-methoxyphenoxy group, a p-chlorophenoxy group and a p-hydroxyphenoxy group. Examples of the arylmercapto group include a phenylmercapto group, a p-methylphenylmercapto group, a p-methoxyphenylmercapto group, a p-chlorophenylmercapto group and a p-hydroxyphenylmercapto group.

Examples of the C₃₋₅ alkylene group in the definition of R¹ and R² include a trimethylene group, a tetramethylene group, a pentamethylene group, a hexamethylene group, a methyltrimethylene group, an ethyltrimethylene group, a dimethyltrimethylene group and a methyltetramethylene group.

Examples of the C₁₋₃ hydroxyalkyl group in the definition of R² and R³ include a hydroxymethyl group, a 2-hydroxyethyl group and a 3-hydroxypropyl group. Examples of the C₅₋₇ cycloalkyl group in the definition of R³ include a cyclopentyl group, a cyclohexyl group and a cycloheptyl group.

In the definition of R³, examples of the C₁₋₅ alkoxy group that is the substituent of a phenyl group include a methoxy group, an ethoxy group, a propoxy group, an isopropoxy group, a butoxy group and a pentyloxy group; examples of the C₂₋₅ (total carbon number) alkoxycarbonyl group include a methoxycarbonyl group, an ethoxycarbonyl group, a propoxycarbonyl group and a butoxycarbonyl group; examples of the C₁₋₃ alkylmercapto group include a methylmercapto group, an ethylmercapto group and a propylmercapto group; examples of the C₁₋₄ alkylamino group include a methylamino group, an ethylamino group, a propylamino group and a butylamino group; and examples of the C₂₋₈ (total carbon number) dialkylamino group include a dimethylamino group, a diethylamino group, a dipropylamino group, and a dibutylamino group.

Examples of the compound (I) that is preferably used as an active ingredient of the agent of the present invention include the following compounds.
3-methyl-1-phenyl-2-pyrazolin-5-one;
3-methyl-1-(2-methylphenyl)-2-pyrazolin-5-one;
3-methyl-1-(3-methylphenyl)-2-pyrazolin-5-one;
3-methyl-1-(4-methylphenyl)-2-pyrazolin-5-one;
3-methyl-1-(3,4-dimethylphenyl)-2-pyrazolin-5-one;
1-(4-ethylphenyl)-3-methyl-2-pyrazolin-5-one;
3-methyl-1-(4-propylphenyl)-2-pyrazolin-5-one;
1-(4-butylphenyl)-3-methyl-2-pyrazolin-5-one;
1-(3-trifluoromethylphenyl)-3-methyl-2-pyrazolin-5-one;
1-(4-trifluoromethylphenyl)-3-methyl-2-pyrazolin-5- one;
1-(2-methoxyphenyl)-3-methyl-2-pyrazolin-5-one;
1-(3-methoxyphenyl)-3-methyl-2-pyrazolin-5-one;
1-(4-methoxyphenyl)-3-methyl-2-pyrazolin-5-one;
1-(3,4-dimethoxyphenyl)-3-methyl-2-pyrazolin-S-one;
1-(4-ethoxyphenyl)-3-methyl-2-pyrazolin-5-one;
3-methyl-1-(4-propoxyphenyl)-2-pyrazolin-5-one;
1-(4-butoxyphenyl)-3-methyl-2-pyrazolin-5-one;
1-(2-chlorophenyl)-3-methyl-2-pyrazolin-5-one;
1-(3-chlorophenyl)-3-methyl-2-pyrazolin-5-one;
1-(4-chlorophenyl)-3-methyl-2-pyrazolin-5-one;
1-(3,4-dichlorophenyl)-3-methyl-2-pyrazolin-5-one;
1-(4-bromophenyl)-3-methyl-2-pyrazolin-5-one;
1-(4-fluorophenyl)-3-methyl-2-pyrazolin-5-one;
1-(3-chloro-4-methylphenyl)-3-methyl-2-pyrazolin-5-one;
1-(3-methylmercaptophenyl)-3-methyl-2-pyrazolin-5-one;
1-(4-methylmercaptophenyl)-3-methyl-2-pyrazolin-5-one;
4-(3-methyl-5-oxo-2-pyrazoline-1-yl) benzoic acid;
1-(4-ethoxycarbonylphenyl)-3-methyl-2-pyrazolin-5-one;
1-(4-nitrophenyl)-3-methyl-2-pyrazolin-5-one;
3-ethyl-1-phenyl-2-pyrazolin-5-one;
1-phenyl-3-propyl-2-pyrazolin-5-one;
1,3-diphenyl-2-pyrazolin-5-one;
3-phenyl-1-(p-tolyl)-2-pyrazolin-5-one;
1-(4-methoxyphenyl)-3-phenyl-2-pyrazolin-5-one;
1-(4-chlorophenyl)-3-phenyl-2-pyrazolin-5-one;
3,4-dimethyl-1-phenyl-2-pyrazolin-5-one;
4-isobutyl-3-methyl-1-phenyl-2-pyrazolin-5-one;
4-(2-hydroxyethyl)-3-methyl-1-phenyl-2-pyrazolin-5-one;
3-methyl-4-phenoxy-1-phenyl-2-pyrazolin-5-one;
3-methyl-4-phenylmercapto-1-phenyl-2-pyrazolin-5-one;
3,3 ', 4,5,6,7-hexahydro-2-phenyl-2H-indazole-3-one;
3-(ethoxycarbonylmethyl)-1-phenyl-2-pyrazolin-5-one;
1-phenyl-2-pyrazolin-5-one;
3-methyl-2-pyrazolin-5-one;
1,3-dimethyl-2-pyrazolin-5-one;
1-ethyl-3-methyl-2-pyrazolin-5-one;
1-butyl-3-methyl-2-pyrazolin-5-one;
1-(2-hydroxyethyl)-3-methyl-2-pyrazolin-5-one;
1-cyclohexyl-3-methyl-2-pyrazolin-5-one;
1-benzyl-3-methyl-2-pyrazolin-5-one;
1-(α-naphthyl)-3-methyl-2-pyrazolin-5-one;
1-methyl-3-phenyl-2-pyrazolin-5-one;
3-methyl-1-(4-methylphenyl)-2-pyrazolin-5-one;
1-(4-butylphenyl)-3-methyl-2-pyrazolin-5-one;
1-(4-methoxyphenyl)-3-methyl-2-pyrazolin-5-one;
1-(4-butoxyphenyl)-3-methyl-2-pyrazolin-5-one;
1-(4-chlorophenyl)-3-methyl-2-pyrazolin-5-one;
1-(4-hydroxyphenyl)-3-methyl-2-pyrazolin-5-one;
1-(3,4-dihydroxyphenyl)-3-methyl-2-pyrazolin-5-one;
1-(2-hydroxyphenyl)-3-methyl-2-pyrazolin-5-one;
1-(3-hydroxyphenyl)-3-methyl-2-pyrazolin-5-one;
1-(4-hydroxyphenyl)-3-methyl-2-pyrazolin-5-one;
1-(3,4-hydroxyphenyl)-3-methyl-2-pyrazolin-5-one;
1-(4-hydroxyphenyl)-3-phenyl-2-pyrazolin-5-one;
1-(4-hydroxymethylphenyl)-3-methyl-2-pyrazolin-5-one;
1-(4-aminophenyl)-3-methyl-2-pyrazolin-5-one;
1-(4-methylaminophenyl)-3-methyl-2-pyrazolin-5-one;
1-(4-ethylaminophenyl)-3-methyl-2-pyrazolin-5-one;
1-(4-butylaminophenyl)-3-methyl-2-pyrazolin-5-one;
1-(4-dimethylaminophenyl)-3-methyl-2-pyrazolin-5-one;
1-(acetamidophenyl)-3-methyl-2-pyrazolin-5-one; and
1-(4-cyanophenyl)-3-methyl-2-pyrazolin-5-one:

As an active ingredient of the agent of the present invention, a compound in a free form represented by the formula (I) as well as a physiologically acceptable salt thereof may also be used. Examples of the physiologically acceptable salt include a salt with mineral acid such as hydrochloric acid, sulfuric acid, hydrogen bromide salt or phosphoric acid; a salt with organic acid such as methanesulfonic acid, p-toluenesulfonic acid, benzenesulfonic acid, acetic acid, glycolic acid, glucuronic acid, maleic acid, fumaric acid, oxalic acid, ascorbic acid, citric acid, salicylic acid, nicotinic acid or tartaric acid; a salt with alkaline metal such as sodium and potassium; a salt with alkaline earth metal such as magnesium or calcium; and a salt with amine such as ammonia, tris(hydroxymethyl)aminomethane, N,N-bis(hydroxyethyl)piperazine, 2-amino-2-methyl-1-propanol, ethanolamine, N-methyl glutamine or L-glutamine. Moreover, a salt with amino acid such as glycine may also be used

As an active ingredient of the agent of the present invention, a hydrate of a compound represented by the above formula (I) or a physiologically acceptable salt thereof, or a solvate of a compound represented by the above formula (I) or a physiologically acceptable salt thereof, may also be used. The type of an organic solvent used to form a solvate is not specifically limited. For example, methanol, ethanol, ether, dioxane or tetrahydrofuran can be exemplified. Furthermore, the compound represented by the above formula (I) may have 1 or more asymmetric carbons depending on the type of a substituent. A stereoisomer such as an optical isomer or a diastereoisomer may be present. As an active ingredient of the medicament of the present invention, a stereoisomer in a pure form, any mixture of stereoisomers, raceme or the like may also be used.

All the compounds represented by the formula (I) are known, and can be easily synthesized by a person skilled in the art using a method described in, for example, JP Patent Publication (Kokoku) No. 5-31523 B (1993).

The dose of the medicament of the present invention is not specifically limited. In general, the dose, as the weight of a compound (active ingredient) represented by the formula (I), is generally, in the case of oral administration, 0.1 to 1000 mg/kg body weight per day, preferably 0.5 to 50 mg/kg body weight per day, and in the case of parenteral administration, 0.01 to 100 mg/kg body weight per day and preferably 0.1 to 10 mg/kg body weight. Preferably, the above dose is administered once a day or administered on several (2 to 3) different occasions per day, and may be appropriately increased or decreased depending on age, pathological conditions or symptoms.

As the agent of the present invention, the compound represented by the above formula (I) or the physiologically acceptable salt thereof, or the hydrate or solvate thereof may be administered as it is. In general, it is preferred that a pharmaceutical composition comprising the above substance which is an active ingredient, and a pharmacologically and pharmaceutically acceptable additive, is prepared and administered.

Examples of pharmacologically and pharmaceutically acceptable additives that can be used herein include excipients, disintegrating agents or disintegrating adjuvant agents, binders, lubricants, coating agents, dye, diluents, base, solubilizing agents or solubilizing adjuvant agents, isotonizing agents, pH regulators, stabilizers, propellants and adhesives.

For a pharmaceutical composition appropriate for oral administration, as additives, there can be used for example excipients such as glucose, lactose, D-mannitol, starch or crystalline cellulose; disintegrating agents or disintegrating adjuvant agents such as carboxymethylcellulose, starch or carboxymetylcellulose calcium; binders such as hydroxypropylcellulose, hydroxypropylmethylcellulose, polyvinylpyrrolidone or gelatine; lubricants such as magnesium stearate or talc; coating agents such as hydroxypropylmethylcellulose, saccharose, polyethylene glycol or titanium oxide; and bases such as petrolatum, liquid paraffin, polyethylene glycol, gelatine, kaolin, glycerin, purified water or hard fat.

For a pharmaceutical composition appropriate for injection or drip, there can be used additives, for example, solubilizing agents or solubilizing adjuvant agents such as distilled water for injection, physiological saline or propylene glycol that can constitute an aqueous injection or an injection that is dissolved when used; isotonizing agents such as glucose, sodium chloride, D-mannitol or glycerine; and pH regulators such as inorganic acid, organic acid, inorganic base and organic base.

The form of the agent of the present invention is not specifically limited, and may be any of the various forms that can be applied by a person skilled in the art. As a medicament appropriate for oral administration, for example, tablets, powders, granules, hard gelatin capsule agents, suppositories or troches can be prepared by using solid pharmaceutical additives, and for example, syrups, emulsions or soft gelatin capsule agents can be prepared by using liquid pharmaceutical additives. Furthermore, as a medicament appropriate for parenteral administration, injections, drops, inhalants, suppositories, percutaneous absorbents, trans-mucosal absorbents or the like can be prepared. A protective agent for the brain (drops) comprising as an active ingredient a compound represented by the above formula (I) has already been clinically used (under the general name "edaravone" and the commercial name "Radicut": produced and marketed by Mitsubishi Pharma Corporation). This commercially available pharmaceutical preparation can be used as it is for the medicament of the present invention.

The agent of the present invention is effective for inhibiting a blood-brain barrier disruption in inflammatory diseases of central nervous system. Examples of such inflammatory diseases of central nervous system include multiple sclerosis, meningitis, cerebritis and brain abscess. Particularly preferred examples of such a disease include multiple sclerosis and meningitis. The agent of the present invention can preferably exhibit an action of inhibiting an increase in permeability of the blood-brain barrier, and an action of inhibiting an increase in the amounts of inflammatory cytokines (TNF-α, IL-1β, etc.) in spinal fluid.

The administration route of the agent of the present invention is not particularly limited, and the agent can be administered orally or parenterally. The administration route of parenteral administration is also not particularly limited, and injection administration can be carried out intravenously, intramuscularly, intradermically or subcutaneously.

The agent of the present invention can preventively be administered before the occurrence of blood-brain barrier disruption. Moreover, the agent of the present invention can be administered to patients suffering from blood-brain barrier disruption for the purpose of preventing deterioration of the symptoms or alleviating the symptoms.

### Examples

The present invention will be described more specifically by the following examples. The scope of the present invention is not limited by the following examples.

### Synthetic Example: Synthesis of 3-methyl-1-phenyl-2-pyrazolin-5-on (hereinafter referred to as edaravone)

13.0 g of ethyl acetoacetate and 10.8 g of phenylhydrazine were added in 50 ml of ethanol, followed by 3 hours of reflux and stirring. After the reaction solution was allowed to stand to cool, the precipitated crystal was collected by filtration, and then recrystalized with ethanol, thereby obtaining 11.3 g of the subject compound in the form of colorless crystals.
Yield: 67%
Melting point: 127.5 to 128.5°C

### Example 1: Evaluation of action of edaravone to inhibit blood-brain barrier disruption, using blood-brain barrier coculture model

### (1) Production of blood-brain barrier coculture model

A blood-brain barrier coculture model was prepared by a method that had previously been reported (Eur. J. Pharm. Sci., 12: 215-222, 2001). Specifically, brain microcapillary endothelial cells and rat astrocytes were isolated, and the two types of cells were cultured on both sides of a Transwell™ filter, so as to prepare a coculture model. The preparation method of such a coculture model will be described below.

A brain capillary was isolated from a bovine brain. The meninx and white matter were eliminated therefrom, and the gray matter was recovered in DMEM (DMEM + S) to which 10% fetal calf serum had been added. Vascular fragments were prepared by homogenizing by hand, using a Wheaton homogenizer, and they were then captured on a 150-µm nylon mesh. The blood vessel was digested with collagenase, trypsin and DNAseI, in DMEM + S at 37°C for 1 hour. The digest was then filtrated through a 200-µm nylon mesh. The brain capillary fractions were resuspended in a frozen mixture (fetal calf serum (FCS) containing 10% DMSO), and the obtained suspension was stored at -80°C.

Astrocytes were isolated from newborn Wister rats (Harlan, Zeist, The Netherlands). The isolated cortex was fragmented, and the fragmented cortex was then incubated together with trypsin-EDTA at 37°C in DMEM. The suspension was filtrated through each of 120-µm and 45-µm nylon meshes. The obtained filtrate was placed in a plastic flask used for tissue culture (Greiner, Alphen a/d Rijn, The Netherlands), and it was then cultured in DMEM + S at 37°C in 10% CO₂ for 3 days. Thereafter, the medium was exchanged with fresh medium every 2 days. At the time when the culture became confluent, it was subcultured at a split ratio of 1 : 3 in a flask covered with poly-D-lysine, using trypsin-EDTA. The culture was allowed to grow until it became confluent again. Subsequently, the astrocyte-conditioned medium was recovered every one day during 2 weeks. The obtained media were stored in frozen mixtures in liquid nitrogen.

The brain capillary was inoculated into a plastic flask used for tissue culture, which had been covered with type IV collagen and fibronectin, and was allowed to adhere to a medium for 4 hours. Thereafter, the medium was exchanged with a growth medium (DMEM + S to which a 50% (v/v) astrocyte-conditioned medium and 125 µg/ml heparin had been added), and growing cells (large quantities of brain capillary endothelial cells and only small quantities of peritheliums) were cultured at 37°C in 10% CO₂.

An *in vitro* blood-brain barrier model was prepared on a Transwell polycarbonate filter covered with type IV collagen (surface area: 0.33 cm²; pore size: 0.4 µm; Coming Costar, Cambridge, MA, U.S.A.). At the time when the culture became approximately 70% confluent (4 or 5 days after inoculation of the brain blood capillary), the brain capillary endothelial cells were treated with trypsin-EDTA for approximately 1 minute. The peritheliums were left in a state of adherence to the lower layer. Astrocytes were inoculated into the bottom of the filter at a density of 45,000 cells/filter, so as to prepare a coculture consisting of the brain capillary endothelial cells and the astrocytes. The astrocytes were allowed to adhere to the bottom of the filter for 10 minutes. Two or three days after such adhesion, the brain capillary endothelial cells were subcultured. The brain capillary endothelial cells were inoculated at a density of 30,000 cells/filter. A coculture consisting of the brain capillary endothelial cells and the astrocytes was cultured in DMEM + S supplemented with 125 µg/ml heparin, for the initial 2 or 3 days, and then cultured in DMEM + S or a differential medium (that is, DMEM + S which had been supplemented with 5 µg/ml apotransferrin, 8 µg/ml putrescine, 2.5 µg/ml sodium selenite, 312.5 µM 8-(4-chlorophenylthio (CPT))-cAMP, 17.5 µM RO-20-1724, and 1 µM all trans-retinoic acid) for the last 2 or 3 days, so that the culture was carried out until the culture product became a tight monolayer. A monolayer consisting of the brain capillary endothelial cells was cultured in the same above manner with the exception that a 50% (v/v) astrocyte-conditioned medium was added to the medium.

A summary of the produced coculture model is shown in the left image of Figure 1. The correlation between the diffusion speed of sodium fluorescein and TEER (transcellular endothelial electrical resistance) in the produced coculture model is shown in the right image of Figure 1. TEER was measured using Millicell-ERS (Catalog No. MERS00001) manufactured by Millipore.

### (2) Effects of TNF-α and IL-1β on TEER

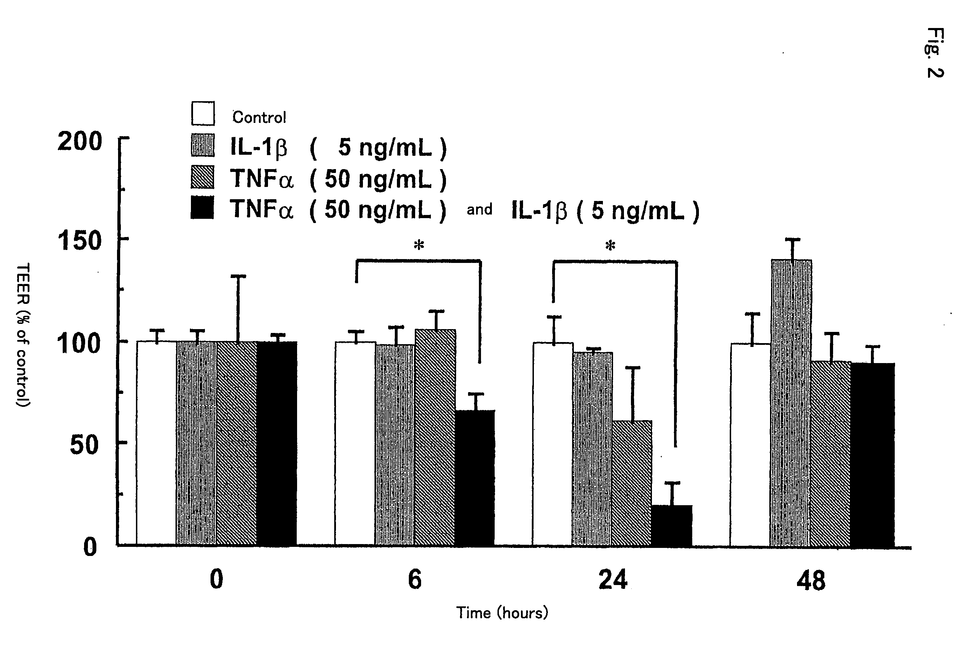

Using the above-described blood-brain barrier coculture model, TNF-α (50 ng/ml) and/or IL-1β (5 ng/ml) were added to the cells, and permeability was then measured as TEER, using Millicell-ERS (Catalog No. MERS00001) manufactured by Millipore. TNF-α and IL-1β were added to the outside of the base of the BBB coculture model in a Transwell™ filter. The results (n = 3, average ± SEM, and *P < 0.05) are shown in Figure 2. As is apparent from the results shown in Figure 2, TEER was significantly reduced by addition of TNF-α (50 ng/ml) and/or IL-1β (5 ng/ml).

### (3) Recovery from TEER reduction by NF-κB inhibitor, iNOS inhibitor and edaravone

TNF-α (50 ng/ml) and/or IL-1β (5 ng/ml) were added to the cells in the presence of BAY11-7082 (10 µM, CALBIOCHEM) or α-MSH (1 µM, Sigma) used as an NF-κB inhibitor, 1400W (0.5 nM, CALBIOCHEM) used as an iNOS inhibitor, or edaravone (10 µM). Thereafter, permeability was measured as TEER, using Millicell-ERS (Catalog No. MERS00001) manufactured by Millipore. In addition, NO in the culture solution was measured by the Griess method.

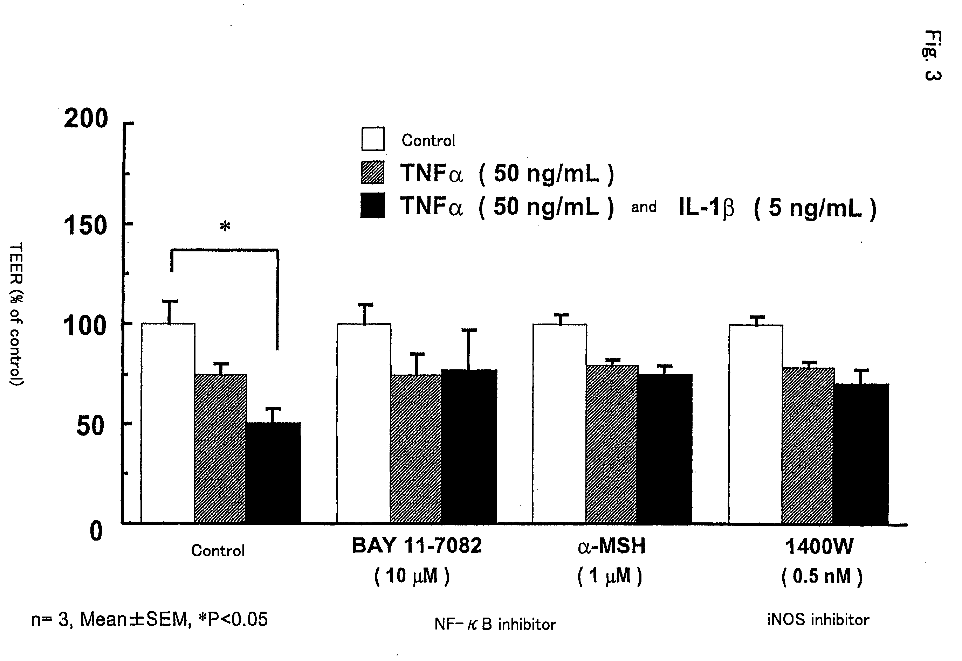

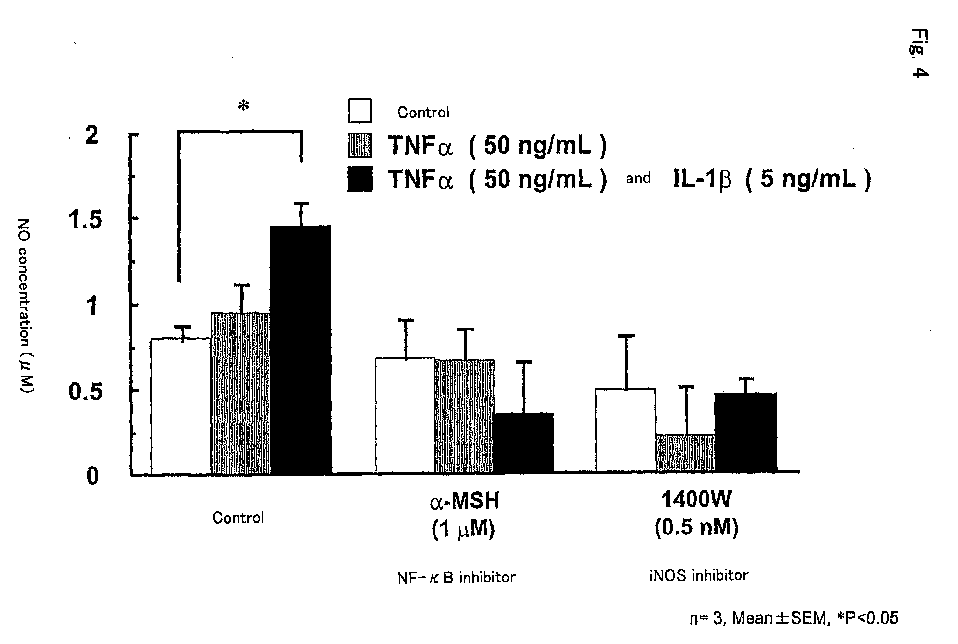

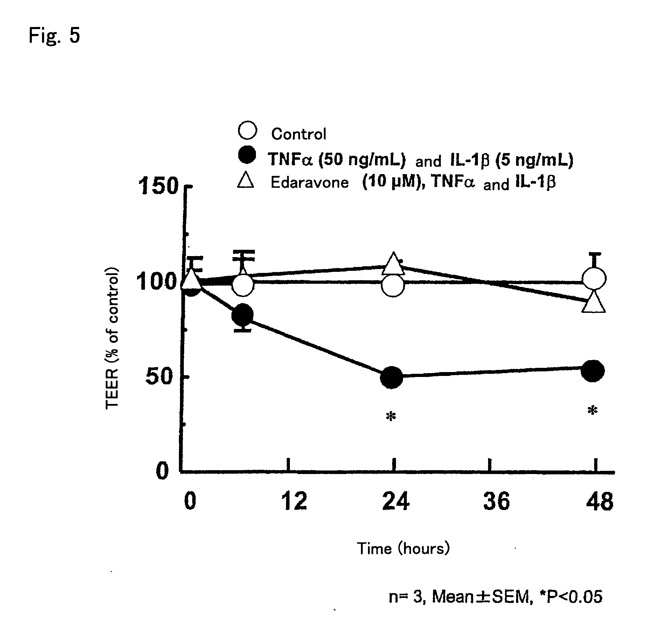

The results (n = 3 in each experiment, average ± SEM, and *P < 0.05) are shown in Figures 3 to 5. Figure 3 shows the effects of the NF-κB inhibitor and iNOS inhibitor on TEER. Figure 4 shows the effects of the NF-κB inhibitor and iNOS inhibitor on the amount of NO produced. Figure 5 shows the effects of edaravone on TEER. As is apparent from the results shown in Figures 3 to 5, reduction in TEER by TNF-α or IL-1β was overcome by addition of the NF-κB inhibitor, iNOS inhibitor, or edaravone.

### Example 2: Evaluation of action of edaravone to inhibit blood-brain barrier disruption, using experimental autoimmune encephalomyelitis (EAE) models

Experimental autoimmune encephalomyelitis (EAE) model rats were prepared by known methods (Int. J. Immunopharmacol, 7: 497-503, 1995). First, an encephalitogenic emulsion consisting of equivalent amounts of Guinea pig spinal cord, sterile phosphate buffered saline (PBS), and Freund's incomplete adjuvant was prepared. Thereafter, 10 mg/ml *Mycobacterium tuberculosis* H₃₇Ra (Difco Laboratories) was added to the emulsion. Rats (inbred male Lewis rats; body weight: 200 to 250 g) were inoculated with 0.1 ml each of the thus obtained emulsion by subcutaneous administration of the emulsion to both plantae of the hind legs thereof, so as to prepare EAE model rats. Control rats were inoculated with an emulsion that did not contain spinal cord.

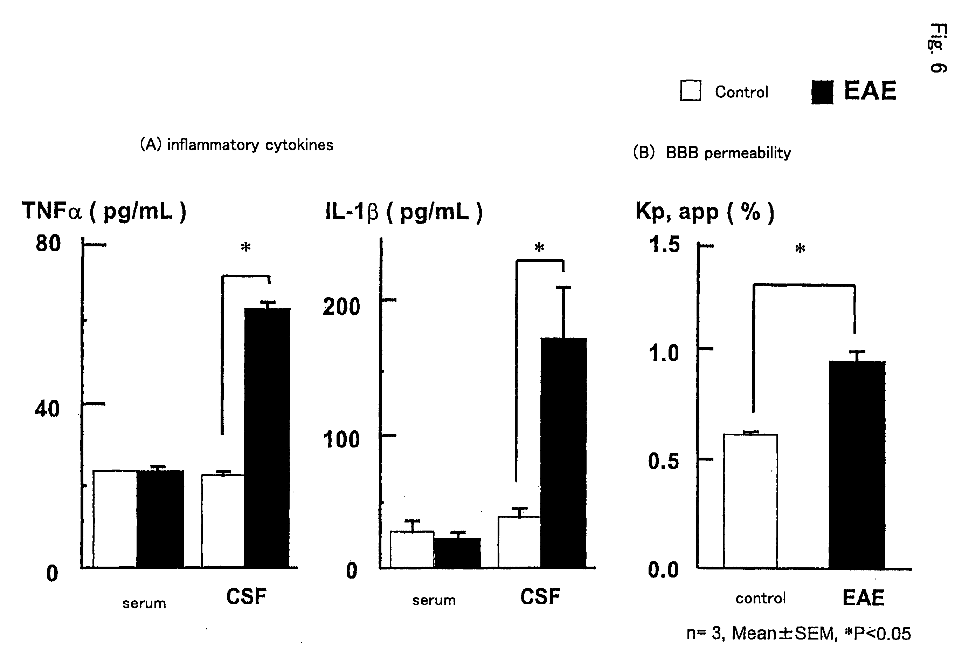

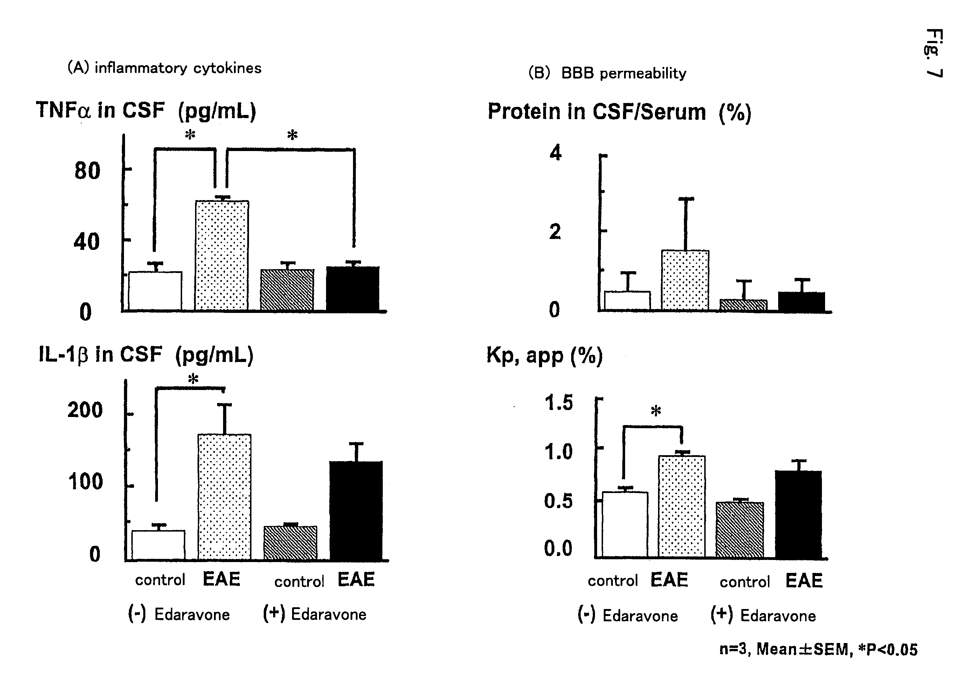

With regard to the control rats and EAE model rats, the concentrations of TNF-α and IL-1β in blood plasma and in spinal fluid, the protein ratio in spinal fluid / blood plasma, and blood-brain barrier permeability (the ratio of the concentration (Kp, app) of sodium fluorescein in brain / blood plasma, 20 minutes after administration by intravenous injection) were measured, in both a case where edaravone was administered or a case where edaravone was not administered. The terms "Kp" and "app" indicate apparent distribution ratios, which are values obtained by dividing the concentration of fluorescein in brain tissues by the concentration thereof in blood plasma after intravenous injection of the fluorescein (wherein the values are multiplied by 100, so as to be expressed on a percentage basis). It is to be noted that the specific gravity of brain tissues is expressed as 1 (because the unit of concentration is expressed as /g tissue weight). The amount of edaravone administered was set at 3 mg/kg/day (intraperitoneal administration). From 10 days after sensitization (administration of an antigen solution), edaravone was administered at intervals of 24 hours for 3 days (that is, 3 administrations at 10 days, 11 days, and 12 days after sensitization). The results (n = 3 in each experiment, average ± SEM, and *P < 0.05 ) are shown in Figures 6 and 7.

As is clear from the results shown in Figures 6 and 7, when compared with the control rats, the EAE model rats exhibited increased concentrations of TNF-α and IL-1β in spinal fluid, increased protein ratio in spinal fluid / blood plasma, and increased blood-brain barrier permeability (the ratio of the concentration of sodium fluorescein in brain / blood plasma; administration was done by intravenous injection). However, as is clear from the results shown in Figure 7, it was demonstrated that these results are inhibited by administration of edaravone.

Moreover, limb neuroparalysis scores were evaluated in a group of EAE model rats to which no agents were administered (control), a group of EAE model rats to which dexamethasone was administered (1 mg/kg/day, intraperitoneal administration), and a group of EAE model rats to which edaravone was administered (3 mg/kg/day, intraperitoneal administration). The limb neuroparalysis scores were evaluated in accordance with the following standards.

**Table 1:**

| Paul's clinical score in EAE rats | |
|---|---|
| Score | Neurological symptoms |
| 0 | Normal |
| 1 | Sagging of tails and locomotor ataxia |
| 2 | Partial paralysis of hind legs |
| 3 | Complete paralysis of hind legs associated with incontinence |
| 4 | Complete paralysis of legs |

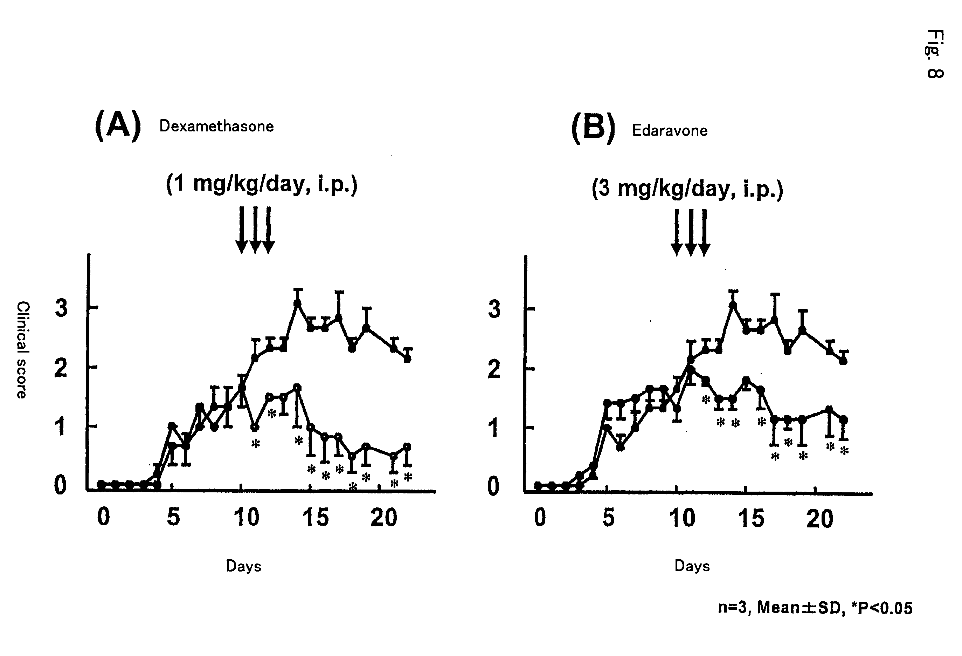

The evaluation results (n = 3 in each experiment, average ± SEM, and *P < 0.05) are shown in Figure 8. As is apparent from the results shown in Figure 8, the limb neuroparalysis scores of the EAE model rats were improved by administration of edaravone, as in the case of administration of dexamethasone.

The aforementioned results of Examples 1 and 2 demonstrate that edaravone protects the blood-brain barrier system, thereby resulting in improvement of neurological symptoms observed in multiple sclerosis models.

### INDUSTRIAL APPLICABILITY

The agent of the present invention is useful for inhibiting blood-brain barrier disruption in inflammatory diseases of central nervous system.

The entire content of Japanese Patent Application No.2003-004813, which the present application claims a priority based on, is incorporated herein by reference as a part of disclosure of the present specification.

## Claims

1. A blood-brain barrier disruption inhibitor which comprises as an active ingredient a pyrazolone derivative represented by the following formula (I) or a physiologically acceptable salt thereof, or a hydrate thereof or a solvate thereof: 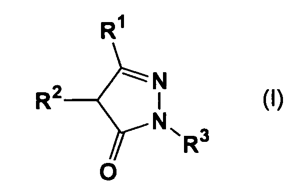 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

2. The blood-brain barrier disruption inhibitor according to claim 1 which has an action of inhibiting increases in permeability of the blood-brain barrier.

3. The blood-brain barrier disruption inhibitor according to claim 1 or 2 which has an action of inhibiting increases in the amount of inflammatory cytokines in spinal fluid.

4. The blood-brain barrier disruption inhibitor according to any of claims 1 to 3 wherein the pyrazolone derivative represented by the formula (I) is 3-methyl-1-phenyl-2-pyrazolin-5-one.

5. A medicament for prevention and/or treatment of multiple sclerosis, meningitis, cerebritis or brain abscess, which comprises as an active ingredient a pyrazolone derivative represented by the above-described formula (I) or a physiologically acceptable salt thereof, or a hydrate thereof or a solvate thereof: 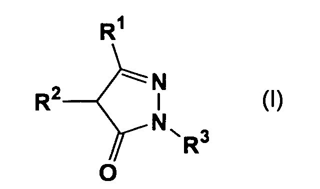 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

6. The medicament according to claim 5 wherein the pyrazolone derivative represented by the formula (I) is 3-methyl-1-phenyl-2-pyrazolin-5-one.

7. A method for inhibiting a blood-brain barrier disruption which comprises a step of administering to mammals such as a human, an effective amount of a pyrazolone derivative represented by the formula (I) or a physiologically acceptable salt thereof, or a hydrate thereof or a solvate thereof: 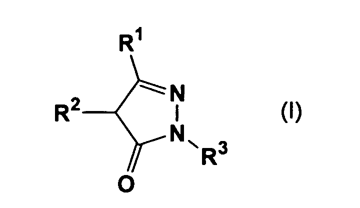 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

8. The method according to claim 7 wherein the blood-brain barrier disruption is inhibited by inhibiting increases in permeability of the blood-brain barrier.

9. The method according to claim 7 or 8 wherein the blood-brain barrier disruption is inhibited by inhibiting increases in the amount of inflammatory cytokines in spinal fluid.

10. The method according to any of claims 7 to 9 wherein the pyrazolone derivative represented by the formula (I) is 3-methyl-1-phenyl-2-pyrazolin-5-one.

11. A method for preventing and/or treating multiple sclerosis, meningitis, cerebritis or brain abscess which comprises a step of administering to mammals such as a human, an effective amount of a pyrazolone derivative represented by the formula (I) or a physiologically acceptable salt thereof, or a hydrate thereof or a solvate thereof: 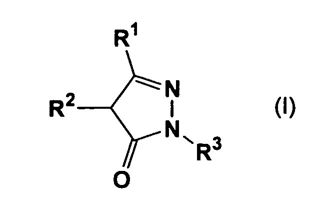 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

12. The method according to claim 11 wherein the pyrazolone derivative represented by the formula (I) is 3-methyl-1-phenyl-2-pyrazolin-5-one.

13. Use of a pyrazolone derivative represented by formula (I) or a physiologically acceptable salt thereof, or a hydrate thereof or a solvate thereof, for the production of a blood-brain barrier disruption inhibitor; 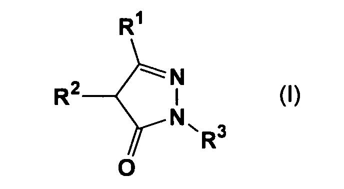 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

14. The use according to claim 13 wherein the blood-brain barrier disruption inhibitor has an action of inhibiting increases in permeability of the blood-brain barrier.

15. The use according to claim 13 or 14 wherein the blood-brain barrier disruption inhibitor has an action of inhibiting increases in the amount of inflammatory cytokines in spinal fluid.

16. The use according to any of claims 13 to 15 wherein the pyrazolone derivative represented by the formula (I) is 3-methyl-1-phenyl-2-pyrazolin-5-one.

17. Use of a pyrazolone derivative represented by formula (I) or a physiologically acceptable salt thereof, or a hydrate thereof or a solvate thereof, for the production of a medicament for prevention and/or treatment of multiple sclerosis, meningitis, cerebritis or brain abscess: 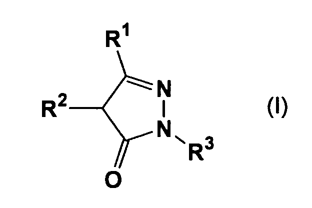 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

18. The use according to claim 17 wherein the pyrazolone derivative represented by the formula (I) is 3-methyl-1-phenyl-2-pyrazolin-5-one.
